(19) Europäisches Patentamt
European Patent Office
Office européen des brevets

(11) Veröffentlichungsnummer: **0 024 329**
**A2**

(12) **EUROPÄISCHE PATENTANMELDUNG**

(21) Anmeldenummer: **80104621.0**

(22) Anmeldetag: **06.08.80**

(51) Int. Cl.³: **C 07 C 149/437**, A 01 N 47/24
// C07C149/14

(30) Priorität: **18.08.79 DE 2933600**

(43) Veröffentlichungstag der Anmeldung: **04.03.81**
**Patentblatt 81/9**

(84) Benannte Vertragsstaaten: **AT BE CH DE FR GB IT LI NL SE**

(71) Anmelder: **BAYER AG, Zentralbereich Patente, Marken und Lizenzen, D-5090 Leverkusen 1, Bayerwerk (DE)**

(72) Erfinder: **Stetter, Jörg, Dr., Gellertweg 4, D-5600 Wuppertal 1 (DE)**
Erfinder: **Homeyer, Bernhard, Dr., Obere Strasse 28, D-5090 Leverkusen 3 (DE)**
Erfinder: **Hammann, Ingeborg, Dr., Belfortstrasse 9, D-5000 Köln 1 (DE)**
Erfinder: **Elbe, Hans-Ludwig, Dr., Dasnoeckel 59, D-5600 Wuppertal 11 (DE)**

(54) **Substituierte 2-Carbamoyloximino-butane, Verfahren zu ihrer Herstellung und ihre Verwendung als Schädlingsbekämpfungsmittel.**

(57) Die vorliegende Erfindung betrifft neue substituierte 2-Carbamoyloximino-butane der allgemeinen Formel (I)

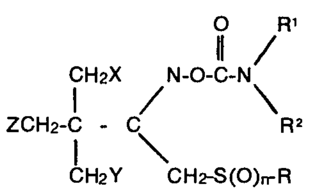

(I)

in welcher R, $R^1$, $R^2$, X, Y, Z die in der Beschreibung angegebene Bedeutung haben sowie Verfahren zu ihrer Herstellung und ihre Verwendung als Schädlingsbekämpfungsmittel.

EP 0 024 329 A2

0024329

BAYER AKTIENGESELLSCHAFT
Zentralbereich
Patente Marken und Lizenzen

5090 Leverkusen-Bayerwerk
Rt/W
Typ Ib

# Substituierte 2-Carbamoyloximino-butane, Verfahren zu ihrer Herstellung und ihre Verwendung als Schädlingsbekämpfungsmittel

Die vorliegende Erfindung betrifft neue substituierte 2-Carbamoyl-oximino-butane, mehrere Verfahren zu ihrer Herstellung und ihre Verwendung als Schädlingsbekämpfungsmittel.

Es ist bereits bekannt geworden, daß Oximcarbamate, wie beispielsweise 1-Cyan-2-methylpropanaldoxim-N-methylcarbamat und 1-Cyan-butanaldoxim-N-methylcarbamat oder 3,3-Dimethyl-2-methylcarbamoyloximino-1-methylthiobutan (Decamox) oder N,N-Dimethyl-α-methylcarbamoyloxyimino-α-methylthio-acetamid (Oxamyl) pestizide Eigenschaften besitzen (vergleiche DE-OS 15 67 142 bzw. DE-OS 22 16 838 bzw. US-Patentschrift 3 530 220 und 3 658 870). Deren Wirkung ist jedoch, insbesondere bei niedrigen Aufwandmengen, nicht immer ganz befriedigend.

Es wurden neue substituierte 2-Carbamoyloximino-butane der allgemeinen Formel (I)

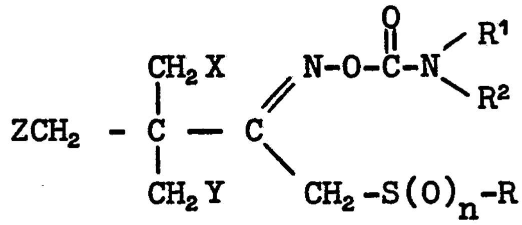

(I)

in welcher

R für Alkyl oder Alkylthioalkyl steht,

$R^1$ für Alkyl, Alkoxyalkyl, Alkenyl oder Alkinyl steht,

$R^2$ für Wasserstoff, Alkyl, oder die Gruppe $-SR^3$ steht, wobei

$R^3$ für Alkyl, Halogenalkyl, gegebenenfalls substituiertes Phenyl, Alkoxycarbonyl, die Gruppierung $-N(CH_3)R^4$, sowie für den gleichen Rest steht, an den die Gruppe - gebunden ist,

$R^4$ für Alkyl, Alkoxycarbonyl oder für gegebenenfalls substituiertes Phenylsulfonyl steht,

X für Wasserstoff oder Halogen steht,

Y für Wasserstoff oder Halogen steht,

Z für Halogen steht und

n für die Zahlen 0, 1 oder 2 steht,

gefunden.

Die Verbindungen der Formel (I) können in der syn- oder anti-Form vorliegen; vorwiegend fallen sie als Gemische beider Formen an.

Weiterhin wurde gefunden, daß die substituierten 2-Carbamoyloximino-butane der Formel (I) zu erhalten sind, wenn man Oxime der Formel (II)

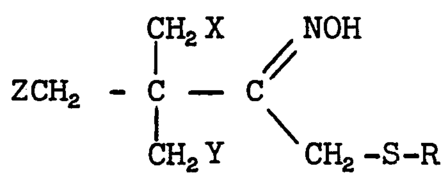

(II)

in welcher

R,X,Y und Z die oben angegebene Bedeutung haben,

a) mit Isocyanaten der Formel (III)

$R^1-N=C=O$ (III)

in welcher

$R^1$ die oben angegebene Bedeutung hat,

in Gegenwart eines Verdünnungsmittels und gegebenenfalls in Gegenwart eines Katalysators umsetzt, oder

b) mit Carbamoylhalogeniden der Formel (IV)

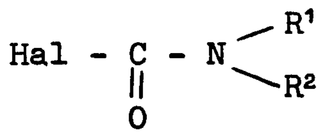

(IV)

in welcher

$R^1$ und $R^2$ die oben angegebene Bedeutung haben und

Hal für Fluor, Chlor oder Brom steht,

in Gegenwart eines Verdünnungsmittels und eines Säurebindemittels umsetzt, oder

c) gegebenenfalls die nach dem Verfahren (a) und (b) erhältlichen Carbamoyloximino-butane der Formel (V)

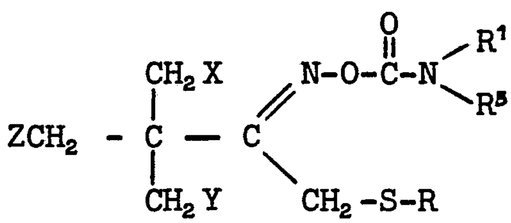

(V)

in welcher

$R, R^1, X, Y$ und Z die oben angegebene Bedeutung haben und

$R^5$ für Wasserstoff oder Alkyl mit 1 bis 4 Kohlenstoffatomen steht,

in üblicher Weise oxidiert, wobei je nach verwendetem Oxidationsmittel Verbindungen der Formel (I) mit n= 1 oder n= 2 erhalten werden, oder

d) gegebenenfalls die nach Verfahren (a) und (c) erhältlichen Carbamoyloximino-butane der Formel (Ia)

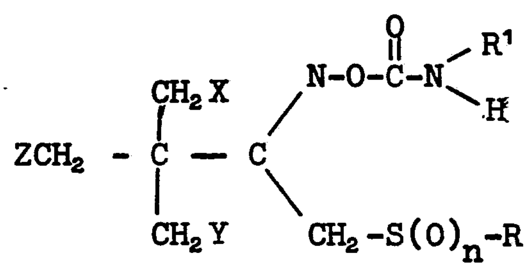

(Ia)

in welcher
$R, R^1$, X,Y,Z und n die oben angegebene Bedeutung haben,

mit Sulfenchloriden bzw. -bromiden der Formel (VI)

$$Hal' - S - R^3 \qquad (VI)$$

in welcher
$R^3$ die oben angegebene Bedeutung hat und
Hal' für Chlor oder Brom steht,

in Gegenwart eines Verdünnungsmittels und eines Säurebindemittels umsetzt.

Die neuen substituierten 2-Carbamoyloximino-butane der Formel (I) eignen sich zur Schädlingsbekämpfung. Sie weisen starke insektizide und akarizide, insbesondere auch wurzelsystemische Eigenschaften auf. Überraschenderweise zeigen die erfindungsgemäßen Verbindungen eine höhere Wirkung als die bekannten Oximcarbamate 1-Cyan-2-methyl-propanaldoxim-N-methyl-carbamat, 1-Cyan-butanaldoxim-N-methylcarbamat, 3,3-Dimethyl-2-methylcarbamoyloximino-1-methylthio-butan und N,N-Dimethyl-α-methylcarbamoyl-oxyimino-α-methylthio-acetamid, welches chemisch und wirkungsmäßig naheliegende Verbindungen sind. Die erfindungsgemäßen Wirkstoffe stellen somit eine Bereicherung der Technik dar.

Die erfindungsgemäßen substituierten 2-Carbamoyloximino-butane sind durch die Formel (I) allgemein definiert. In dieser Formel steht R vorzugsweise für geradkettiges

oder verzweigtes Alkyl mit 1 bis 4 Kohlenstoffatomen oder für Alkylthioalkyl mit 1 bis 4 Kohlenstoffatomen in jedem Alkylteil. $\underline{R}^1$ steht vorzugsweise für geradkettiges oder verzweigtes Alkyl mit 1 bis 4 Kohlenstoffatomen,Alkoxyalkyl mit 1 bis 4 Kohlenstoffatomen in jedem Alkylteil, Alkenyl und Alkinyl mit jeweils 2 bis 4 Kohlenstoffatomen. $\underline{R}^2$ steht vorzugsweise für Wasserstoff, Alkyl mit 1 bis 4 Kohlenstoffatomen und die Gruppe $-SR^3$. $\underline{R}^3$ steht vorzugsweise für geradkettiges oder verzweigtes Alkyl mit 1 bis 4 Kohlenstoffatomen, Halogenalkyl mit 1 bis 2 Kohlenstoff- und bis zu 5 Halogenatomen, wie insbesondere Fluor- und Chloratomen, gegebenenfalls substituiertes Phenyl, wobei als Substituenten vorzugsweise genannt seien: Halogen, insbesondere Fluor, Chlor oder Brom, Alkyl mit 1 bis 2 Kohlenstoffatomen und Halogenalkyl mit 1 bis 2 Kohlenstoff- und bis zu 5 Halogenatomen, wie insbesondere Fluor- und Chloratomen, sowie vorzugsweise für Alkoxycarbonyl mit 1 bis 4 Kohlenstoffatomen im Alkylteil. $\underline{R}^3$ steht außerdem vorzugsweise für die Grüppierung $-N(CH_3)R^4$, sowie für den gleichen Rest, an den die Gruppe $-SR^3$ gebunden ist. $\underline{R}^4$ steht vorzugsweise für Alkyl mit 1 bis 4 Kohlenstoffatomen, Alkoxycarbonyl mit 1 bis 4 Kohlenstoffatomen im Alkylteil sowie für gegebenenfalls substituiertes Phenylsulfonyl, wobei als Phenylsubstituenten vorzugsweise die bei $\underline{R}^3$ bereits genannten Phenylsubstituenten infrage kommen. $\underline{X}$ steht vorzugsweise für Wasserstoff, Fluor, Chlor oder Brom. $\underline{Y}$ steht vorzugsweise für Wasserstoff, Fluor, Chlor oder Brom. $\underline{Z}$ steht vorzugsweise für Fluor, Chlor oder Brom. Der Index $\underline{n}$ steht vorzugsweise für die Zahlen 0, 1 oder 2.

Ganz besonders bevorzugt sind diejenigen substituierten 2-Carbamoyloximino-butane der Formel (I), in denen $\underline{R}$ für Methyl, Ethyl, Methylthiomethyl oder Ethylthiomethyl steht; $\underline{R}^1$ für Methyl, Ethyl, Methoxymethyl oder Allyl steht; $\underline{R}^2$ für Wasserstoff, Methyl, Ethyl, oder die Gruppe

$-SR^3$ steht; $\underline{R}^3$ für Methyl, Trifluormethyl, Chlordifluormethyl, Dichlorfluormethyl, Trichlormethyl, gegebenenfalls durch Chlor oder Trifluormethyl substituiertes Phenyl, Methoxycarbonyl, Ethoxycarbonyl und die Gruppierung $-N(CH_3)R^4$ steht, oder $\underline{R}^3$ für den gleichen Rest steht, an den die Gruppe $-SR^3$ gebunden ist; $\underline{R}^4$ für Methyl, Ethyl, Methoxycarbonyl, Ethoxycarbonyl und gegebenenfalls durch Chlor oder Methyl substituiertes Phenylsulfonyl steht ; $\underline{X}$,$\underline{Y}$,$\underline{Z}$ und der Index $\underline{n}$ die oben angegebene Bedeutung haben.

Im einzelnen seien außer den bei den Herstellungsbeispielen genannten Beispielen folgende Verbindungen genannt:

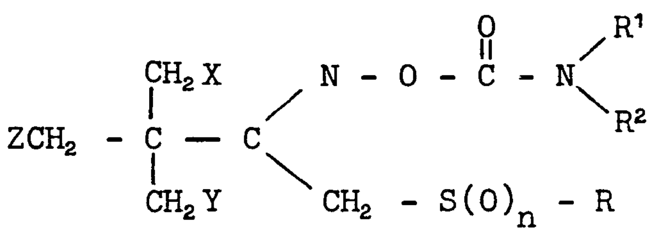

(I)

| X | Y | Z | R | $R^1$ | $R^2$ | n |
|---|---|---|---|---|---|---|
| H | H | F | $CH_3$ | $CH_3$ | $-SCH_3$ | 0 |
| H | H | Cl | $CH_3$ | $CH_3$ | $-SCH_3$ | 0 |
| H | H | Br | $CH_3$ | $CH_3$ | $-SCH_3$ | 0 |
| H | F | F | $CH_3$ | $CH_3$ | $-SCH_3$ | 0 |
| H | Cl | Cl | $CH_3$ | $CH_3$ | $-SCH_3$ | 0 |
| H | Br | Br | $CH_3$ | $CH_3$ | $-SCH_3$ | 0 |
| H | H | F | $CH_3$ | $CH_3$ | 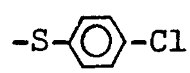 | 0 |
| H | H | Cl | $CH_3$ | $CH_3$ | 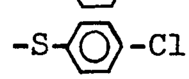 | 0 |
| H | H | Br | $CH_3$ | $CH_3$ | 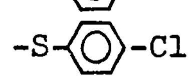 | 0 |
| H | F | F | $CH_3$ | $CH_3$ | 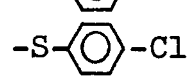 | 0 |

| X | Y | Z | R | $R^1$ | $R^2$ | n |
|---|---|---|---|---|---|---|
| H | Cl | Cl | $CH_3$ | $CH_3$ | 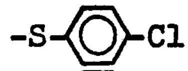 | 0 |
| H | Br | Br | $CH_3$ | $CH_3$ | 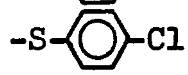 | 0 |
| H | H | F | $CH_3$ | $CH_3$ | $-S-CCl_3$ | 0 |
| H | H | Cl | $CH_3$ | $CH_3$ | $-S-CCl_3$ | 0 |
| H | H | Br | $CH_3$ | $CH_3$ | $-S-CCl_3$ | 0 |
| H | F | F | $CH_3$ | $CH_3$ | $-S-CCl_3$ | 0 |
| H | Cl | Cl | $CH_3$ | $CH_3$ | $-S-CCl_3$ | 0 |
| H | Br | Br | $CH_3$ | $CH_3$ | $-S-CCl_3$ | 0 |
| H | H | F | $CH_3$ | $CH_3$ | $-S-\underset{\substack{\vert\\CH_3}}{N}-COOC_2H_5$ | 0 |
| H | H | Cl | $CH_3$ | $CH_3$ | $-S-\underset{\substack{\vert\\CH_3}}{N}-COOC_2H_5$ | 0 |
| H | H | Br | $CH_3$ | $CH_3$ | $-S-\underset{\substack{\vert\\CH_3}}{N}-COOC_2H_5$ | 0 |
| H | H | H | $CH_3$ | $CH_3$ | H | 0 |
| H | Cl | Cl | $CH_3$ | $CH_3$ | H | 0 |
| H | Br | Br | $CH_3$ | $CH_3$ | H | 0 |
| H | H | Cl | $CH_3$ | $CH_3$ | H | 0 |
| H | H | Br | $CH_3$ | $CH_3$ | H | 0 |
| H | H | F | $CH_3$ | $CH_3$ | H | 1 |
| H | F | F | $CH_3$ | $CH_3$ | H | 1 |
| H | H | F | $CH_3$ | $CH_3$ | H | 2 |
| H | F | F | $CH_3$ | $CH_3$ | H | 2 |
| F | F | F | $CH_3$ | $CH_3$ | H | 0 |
| F | F | F | $CH_3$ | $CH_3$ | H | 1 |
| F | F | F | $CH_3$ | $CH_3$ | H | 2 |

| X | Y | Z | R | $R^1$ | $R^2$ | n |
|---|---|---|---|---|---|---|
| H | H | F | $CH_3$ | $CH_3$ | 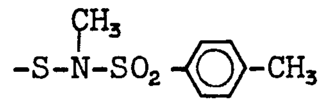 | 0 |
| H | F | F | $CH_3$ | $CH_3$ | 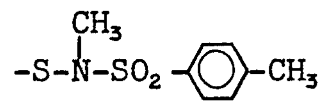 | 0 |
| H | H | F | $CH_3$ | $CH_3$ | $-S-N(CH_3)_2$ | 0 |
| H | F | F | $CH_3$ | $CH_3$ | $-S-N(CH_3)_2$ | 0 |
| H | F | F | $CH_3$ | $CH_3$ | dimer | 0 |

Verwendet man beispielsweise 3,3-Dimethyl-4-fluor-1-methylthio-2-hydroximino-butan und Methylisocyanat als Ausgangsstoffe, so kann der Reaktionsablauf durch das folgende Formelschema wiedergegeben werden(Verfahren a):

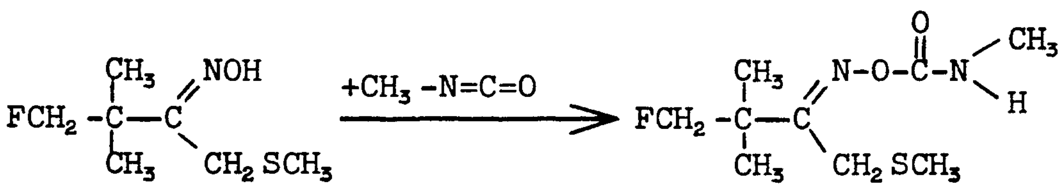


Verwendet man beispielsweise 3,3-Dimethyl-4-fluor-1-methylthio-2-hydroximino-butan und N-Methyl-N-trichlormethyl-2-mercapto-carbamoylfluorid als Ausgangsstoffe, so kann der Reaktionsablauf durch das folgende Formelschema wiedergegeben werden (Verfahren b):

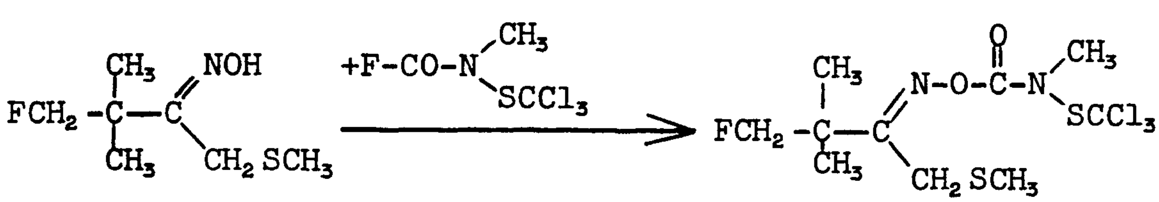

Verwendet man beispielsweise 2 Mol 3,3-Dimethyl-4-fluor-1-methylthio-2-hydroximino-butan und 1 Mol N,N'-Bis-(fluorcarbonyl)-thio-bis-methylamin als Ausgangsstoffe, so kann der Reaktionsablauf durch das folgende Formelschema wiedergegeben werden (Verfahren b):

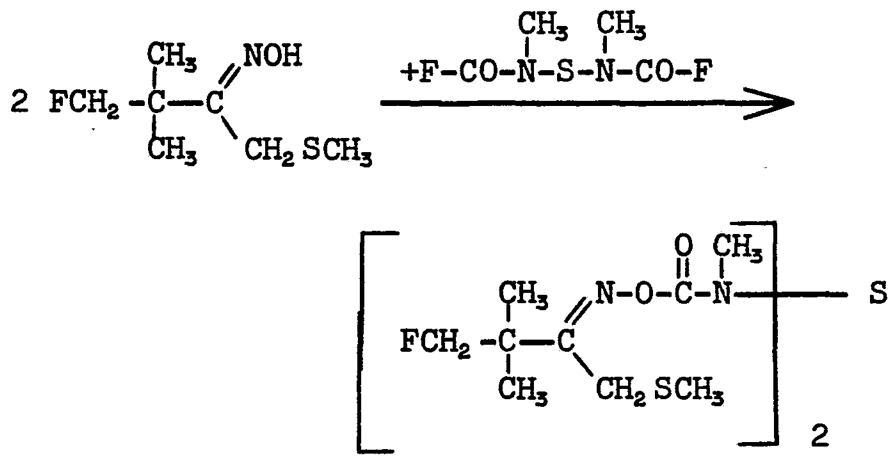

Verwendet man beispielsweise 3,3-Dimethyl-4-fluor-2-methylcarbamoyl-oximino-1-methylthio-butan als Ausgangsstoff und Wasserstoffperoxid als Oxidationsmittel, so kann der Reaktionsablauf durch das folgende Formelschema wiedergegeben werden (Verfahren c):

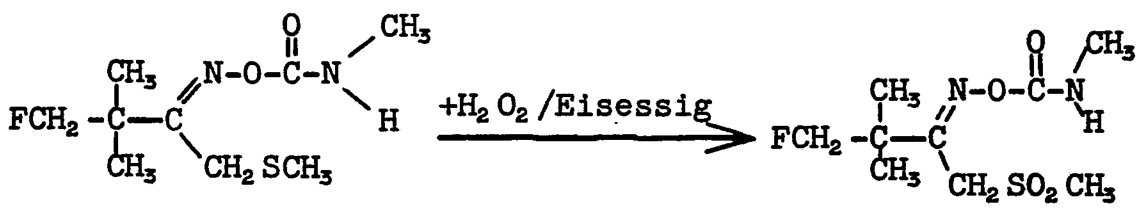


Verwendet man beispielsweise 3,3-Dimethyl-4-fluor-2-methyl-carbamoyl-oximino-1-methylthio-butan und 4-Chlorphenyl-sulfenylchlorid als Ausgangsstoffe, so kann der Reaktionsablauf durch das folgende Formelschema wiedergegeben werden (Verfahren d):

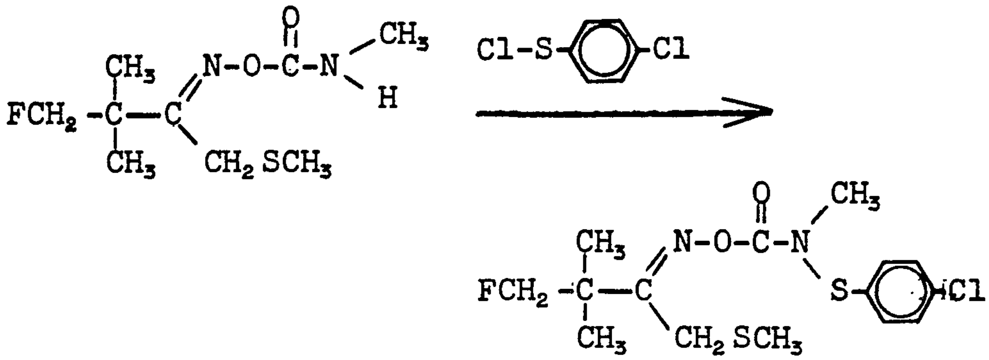

Die für die Verfahren (a) und (b) als Ausgangsstoffe zu verwendenden Oxime sind durch die Formel (II) allgemein definiert. In dieser Formel stehen R, X, Y, Z und n vorzugsweise für die Reste, die bei den Verbindungen der Formel (I) bereits vorzugsweise genannt wurden.

Die Oxime der Formel (II) sind noch nicht bekannt; sie könne jedoch auf bekannte Art und Weise erhalten werden, indem man Ketone der Formel

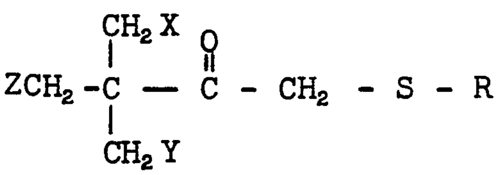

(VII)

in welcher

R,X,Y und Z die oben angegebene Bedeutung haben,

mit Hydroxylamin in Gegenwart eines Lösungsmittels, vorzugsweise Alkohole bzw. wäßrige Alkohole, bei Temperaturen zwischen 20 und 100°C, vorzugsweise zwischen 50 und 80°C, umsetzt. Dabei wird das Hydroxylamin vorzugsweise in Form seiner Salze, insbesondere als Hydrochlorid, in Gegenwart eines Säurebindemittels, wie beispielsweise Natriumcarbonat, eingesetzt. Die Isolierung der Verbindungen der Formel (II) erfolgt dadurch, daß man das während der Umsetzung entstehende Produkt nach Abdestillieren des Lösungsmittels nach üblichen Methoden aufarbeitet. (vergleiche auch die Herstellungsbeispiele).

Die Ketone der Formel (VII) können erhalten werden,indem man α-Halogen-ketone der Formel

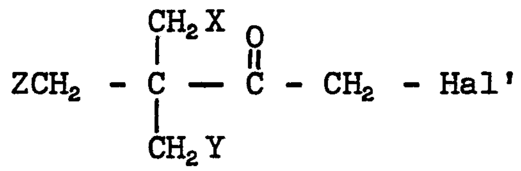

(VIII)

in welcher

Hal',X,Y und Z die oben angegebene Bedeutung haben,

mit Mercaptanen der Formel

H - S - R (IX)

in welcher

R die oben angegebene Bedeutung hat,

in Gegenwart eines inerten organischen Lösungsmittels, wie beispielsweise Methanol, und in Gegenwart einer Base, wie beispielsweise Nartiummethylat, bei Temperaturen zwischen 0 und 100°C umsetzt (vergleiche auch die Herstellungsbeispiele).

Die α-Halogenketone der Formel (VIII) sind teilweise bekannt (vergleiche z.B. DE-OS 26 32 603 [LeA 17 273], teilweise sind sie Gegenstand einer eigenen Anmeldung, die noch nicht bekannt ist (vergleiche deutsche Patentanmeldung P 29 18 894 vom 10.05.1979 [LeA 19 618]). Sie werden erhalten, indem man Butan-Derivate der Formel

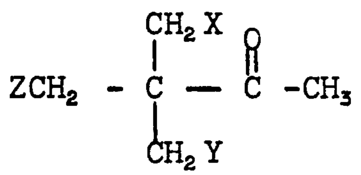 (X)

in welcher

X,Y und Z die oben angegebene Bedeutung haben,

mit Chlor oder Brom in Gegenwart eines inerten organischen Lösungsmittels, wie beispielsweise Ether oder chlorierte Kohlenwasserstoffe, bei Raumtemperatur versetzt (vergleiche auch die Herstellungsbeispiele), oder mit üblichen Chlorierungsmittels, wie beispielsweise Sulfurylchlorid, bei 20 bis 60°C umsetzt.

Die außerdem für das Verfahren (a) als Ausgangsstoffe benötigten Isocyanate sind durch die Formel (III) allgemein definiert. In dieser Formel steht $\underline{R}^1$ vorzugsweise für die Reste, die bei den Verbindungen der Formel (I) bereits vorzugsweise genannt wurden.

Die Isocyanate der Formel (III) sind bekannt oder lassen sich nach allgemein üblichen und bekannten Verfahren herstellen.

Die außerdem für das Verfahren (b) als Ausgangsstoffe benötigten Carbamoylhalogenide sind durch die Formel (IV) allgemein definiert. In dieser Formel stehen $\underline{R}^1$ und $\underline{R}^2$ vorzugsweise für die Reste, die bei den Verbindungen der Formel (I) bereits vorzugsweise genannt wurden.

Die Carbamoylhalogenide der Formel (IV) sind bekannt oder lassen sich nach allgemein üblichen und bekannten Verfahren herstellen, z.B. durch Umsetzung von Aminen mit Phosgen (diese Verfahren sind aus den allgemeinen Lehrbüchern der organischen Chemie bekannt) oder durch Umsetzung der entsprechenden Carbamidsäurehalogenide mit entsprechenden Sulfenchloriden (vergleiche hierzu auch die Angaben in DE-AS 1 297 095, DE-OS 23 57 930 und 24 09 463 sowie US-Patentschrift 3 939 192).

Die weiterhin für das Verfahren (d) als Ausgangsstoffe benötigten Sulfenchloride bzw. -bromide sind durch die Formel (VI) allgemein definiert. In dieser Formel steht $R^3$ vorzugsweise für die Reste, die bei den Verbindungen der Formel (I) bereits vorzugsweise genannt wurden.

Die Sulfenchloride bzw. -bromide sind allgemein bekannte Verbindungen der organischen Chemie.

Als Verdünnungsmittel kommen für die Umsetzung gemäß Verfahrensvarianten (a), (b) und (d) vorzugsweise alle inerten organischen Lösungsmittel infrage. Hierzu gehören vorzugsweise Ketone, wie Diethylketon, insbesondere Aceton und Methylethylketon; Nitrile, wie Propionitril, insbesondere Acetonitril; Alkohole, wie Ethanol oder Isopropanol; Ether, wie Tetrahydrofuran oder Dioxan; Formamide, wie insbesondere Dimethylformamid und halogenierte Kohlenwasserstoffe, wie Methylenchlorid, Tetrachlorkohlenstoff oder Chloroform.

Wird die Umsetzung nach Verfahren (b) und (d) in Gegenwart eines Säurebinders vorgenommen, so kann man alle üblicherweise verwendbaren anorganischen und organischen Säurebinder zugeben. Hierzu gehören vorzugsweise Natriumcarbonat, Kaliumcarbonat und Natriumhydrogencarbonat, ferner niedere tertiäre Alkylamine, Cycloalkylamine oder Aralkylamine, wie beispielsweise Triethylamin, N,N-Dimethyl-benzylamin, Dicyclohexylamin, weiterhin Pyridin und Diazabicyclooctan.

Als Katalysatoren können beim Verfahren (a) vorzugsweise verwendet werden: tertiäre Basen, wie Triethylamin, Pyridin, Zinn-organische Verbindungen, Dibutylzinndilaurat.

Die Reaktionstemperaturen können bei der Durchführung des Verfahrens (a) in einem größeren Bereich variiert werden. Im allgemeinen arbeitet man zwischen 0 und 100°C, vorzugsweise zwischen 20 und 85°C.

Bei der Durchführung des Verfahrens (a) setzt man auf 1 Mol der Verbindung der Formel (II) 1 bis 2 Mol Isocyanat der Formel (III) ein. Zur Isolierung der Verbindungen der Formel (I) wird das Lösungsmittel abdestilliert und der Rückstand nach üblichen Methoden aufgearbeitet.

Die Reaktionstemperaturen können bei der Durchführung des Verfahrens (b) in einem größeren Bereich variiert werden. Im allgemeinen arbeitet man zwischen 0 und 100°C, vorzugsweise zwischen 10 und 80°C.

Bei der Durchführung des Verfahrens (b) setzt man vorzugsweise auf 1 Mol der Verbindung der Formel (II) 1 bis 2 Mol, für den Fall eines dimeren Produktes 0,5 Mol, an Carbamoylhalogenid der Formel (IV) und 1 bis 2 Mol Säurebinder ein. Die Isolierung der Verbindung der Formel (I) erfolgt in allgemein üblicher und bekannter Weise.

Für das Verfahren (c) kommen als Oxidationsmittel alle üblicherweise verwendbaren anorganischen und organischen Oxidationsmittel in Betracht, wie Chlor in Wasser; Persäuren, beispielsweise Metachlorperbenzoesäure; Wasserstoffperoxid in Eisessig oder Methanol, Kaliumpermanganat und Chromsäure.

Die Reaktionstemperaturen können bei der Oxidation gemäß Verfahren (c) in einem größeren Bereich variiert werden. Im allgemeinen arbeitet man zwischen etwa -30 bis+100°C, vorzugsweise bei -10 bis+80°C.

Bei der Durchführung der Oxidation gemäß Verfahren (c) setzt man auf 1 Mol der Verbindung der Formel (V) etwa 1 bis 4 Mol Oxidationsmittel ein. Bei der Anwendung von 1 Mol Oxidationsmittel, wie Metachlorperbenzoesäure in Methylenchlorid oder Wasserstoffperoxid in Acetanhydrid bei Temperaturen zwischen -10 bis +10°C entstehen vorzugsweise die erfindungsgemäßen Verbindungen der Formel (I) mit n= 1. Bei Ueberschuß an Oxidationsmittel und höheren Temperaturen (10 bis 80°C) entstehen vorzugsweise die erfindungsgemäßen Verbindungen der Formel (I) mit n=2.

Zur Isolierung der Oxidationsprodukte wird das Reaktionsgemisch entweder auf Eiswasser gegeben und abgesaugt, der zurückbleibende Niederschlag wird gegebenenfalls gewaschen und getrocknet, oder die Reaktionslösung wird auf pH 7 bis 8 gestellt, mit einem organischen Lösungsmittel extrahiert und die extrahierte Phase getrocknet und das Lösungsmittel abdestilliert. Die Reaktionsprodukte können in beiden Fällen durch Umkristallisation oder Säulenchromatographie gereinigt werden.

Die Reaktionstemperaturen können bei der Durchführung des Verfahrens (d) in einem größeren Bereich variiert werden. Im allgemeinen arbeitet man zwischen 0 und 100°C, vorzugsweise zwischen 10 und 50°C.

Bei der Durchführung des Verfahrens (d) werden die Ausgangsstoffe vorzugsweise in molaren Mengen eingesetzt. Die Isolierung der Verbindungen der Formel (I) erfolgt nach üblichen Methoden.

Zum Teil ist es auch möglich die einzelnen Stufen der Vorprodukte zur Herstellung der Oxime der Formel (II) sowie deren Umsetzung zu den erfindungsgemäßen Stoffen ohne Isolierung der jeweiligen Zwischenprodukte in einer sogenannten Eintopfreaktion durchzuführen.

Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp.,Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp. Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp.,

Die Wirkstoffe eignen sich bei guter Pflanzenverträglichkeit und günstiger Warmblütertoxizität zur Bekämpfung von tierischen Schädlingen, insbesondere Insekten und Spinnentieren die in der Landwirtschaft, in Forsten, im Vorrats- und Materialschutz sowie auf dem Hygienesektor vorkommen. Sie sind gegen normal sensible und resistente Arten sowie gegen alle oder einezlne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophaus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips fermoralis, Thrips tabaci.

Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Aus der Ordnung der Arachnida z.B. Scorpio maurus, Latrodectus mactans.

Aus der Ordnung der Acarina z.B. Acarus siro, Argas spp., Crnithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff

und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

0024329

Die Anwendung der erfindungsgemäßen Wirkstoffe erfolgt in Form ihrer handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,000001 bis zu 100Gew.-% Wirkstoff, vorzugsweise zwischen 0,01 und 10 Gew.-% liegen.

Die Anwendung geschieht in einer den Anwendungsformen angepaßten üblichen Weise.
Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Beispiel A

Grenzkonzentrations-Test / Wurzelsystemische Wirkung

Testinsekt: Myzus persicae
Lösungsmittel: 3 Gewichtsteile Aceton
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 1.

0024329

## Beispiel B

Grenzkonzentrations-Test / Wurzelsystemische Wirkung

Testinsekt: Phaedon cochleariae -Larven
Lösungsmittel: 3 Gewichtsteile Aceton
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 1.

0024329

Beispiel C

Phaedon-Larven-Test

Lösungsmittel: 3 Gewichtsteile Dimethylformamid
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käfer-Larven abgetötet wurden; 0 % bedeutet, daß keine Käfer-Larven abgetötet wurden.

Bei diesem Test zeigen z.B. die folgenden Verbindungen der Herstellungsbeispiele überlegene Wirksamkeit gegenüber dem Stand der Technik: 1.

**Beispiel D**

Tetranychus-Test (resistent)

Lösungsmittel: 3 Gewichtsteile Dimethylformamid
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris),die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

Bei diesem Test zeigen z.B. die folgenden Verbindungen der Herstellungsbeispiele überlegene Wirksamkeit gegenüber dem Stand der Technik: 1.

Beispiel 1

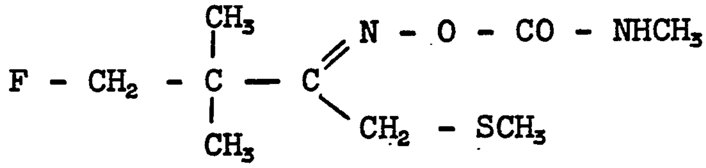

(Verfahren a)

78g (0,43 Mol) 3,3-Dimethyl-4-fluor-1-methylthio-hydroximinobutan werden in 300 ml Methylenchlorid gelöst und tropfenweise mit 26g (0,46 Mol) Methylsiocyanat versetzt. Die Temperatur der Reaktionslösung steigt hierbei auf ca.35°C an.

Nach 5-stündigem Rühren bei Zimmertemperatur wird das Lösungsmittel im Vakuum abgezogen. Nach Verreiben mit Diisopropylether erhält man 65g (64 % der Theorie) 3,3-Dimethyl-4-fluor-2-methylcarbamoyloximino-1-methylthio-butan als farblose Kristalle mit einem Schmelzpunkt von 50-53°C.

Herstellung der Vorprodukte

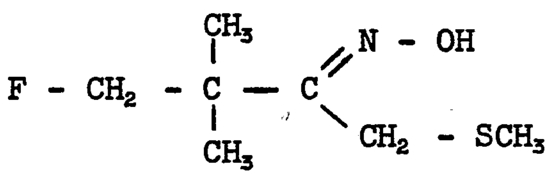

99g (0,6 Mol) 3,3-Dimethyl-4-fluor-1-methylthio-butan-2-on, 46g (0,66 Mol) Hydroxylaminhydrochlorid und 66,7g (0,66 Mol) Triethylamin werden in 300 ml Ethanol 6 Stunden unter Rückfluß erhitzt. Anschließend wird das Lösungsmittel im Vakuum entfernt, der Rückstand zwischen Methylenchlorid und Wasser verteilt, die Methylenchloridphase abgetrennt und mit Natriumsulfat getrocknet. Nach Abdestillieren des Lösungsmittels wird der Rückstand im Vakuum destilliert. Man erhält 80g (75%der Theorie)

3,3-Dimethyl-4-fluor-1-methylthio-2-hydroximino-butan vom Siedepunkt 133°C/13mm Hg-Säule.

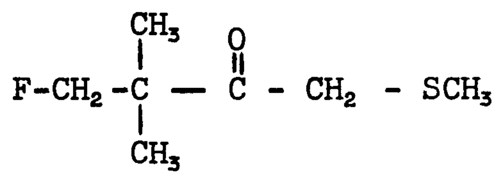

In 150 ml Methanol wird bei -20°C 40g (0,83 Mol) Methylmercaptan eingeleitet. Anschließend wird bei gleicher Temperatur eine Lösung von 45g (0,83 Mol) Natriummethylat in 200 ml Methanol zugetropft. Bei 0°C werden nun 154g (0,78 Mol) 1-Brom-3,3-dimethyl-4-fluor-butan-2-on zugetropft. Das Gemisch wird noch 12 Stunden bei 20°C gerührt, der anorganische Niederschlag abfiltriert und das Filtrat destilliert. Man erhält 99g (77 % der Theorie) 3,3-Dimethyl-4-fluor-1-methylthio-butan-2-on vom Siedepunkt 86°C/10 mm Hg-Säule.

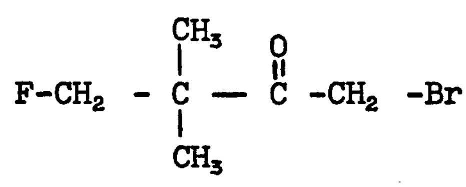

In eine Mischung aus 354g (3 Mol) 3,3-Dimethyl-4-fluor-2-butanon und 2000 ml Ether werden bei 20°C bis 30°C unter Kühlen und Rühren 480g Brom langsam eingetropft. Die gelbliche Lösung wird noch 1 Stunde bei 20° C nachgerührt und anschließend vorsichtig mit 500 ml Wasser versetzt. Die Etherphase wird abgetrennt, mehrmals mit Wasser gewaschen und über Natriumsulfat getrocknet. Nach Abdestillieren des Lösungsmittels wird der Rückstand im Wasserstrahlvakuum destilliert. Man erhält 472g (80% der Theorie) 1-Brom-3,3-dimethyl-4-fluor-2-butanon vom Siedepunkt 80-90°C/11 mmHg-Säule.

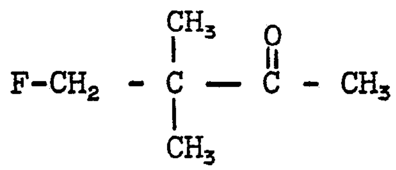

Zu der in einem Dreihals-Rührkolben mit absteigendem Kühler befindlichen Suspension von 23,2g (0,4 Mol) trockenem Kaliumfluorid in 400 ml destilliertem Tetraethylenglykol werden bei 160°C und 20 mbar 38,8g (0,2 Mol) 2,2-Dimethyl-2-oxobutyl-methansulfonat im Verlauf von 2 Stunden zugetropft und 2 weitere Stunden nachgerührt. An einem absteigenden Kondensator und in einer nachgeschalteten Tiefkühlfalle wird das herausdestillierte Reaktionsprodukt kondensiert und gesammelt. Man erhält 20,9g (89 % der Theorie) 3,3-Dimethyl-4-fluor-2-butanon, vom Siedepunkt 130-134°C.

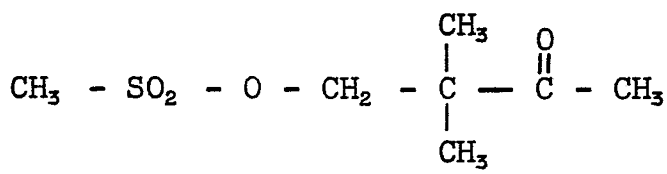

232g (2 Mol) 3,3-Dimethyl-4-hydroxy-2-butanon (z. Herstellung vgl. Beilstein <u>H 1</u> E III 3239, IV 4030 und Bull.Soc.Chim.France <u>1964</u>, 2849) werden in 700 ml absolutem Pyridin bei 0 bis 5°C mit 229g (2 Mol) Methansulfochlorid umgesetzt. Nach 12 Stunden Stehen bei 20°C wird mit Methylenchlorid verdünnt und mit Eiswasser ausgeschüttelt. Die organische Phase wird getrocknet, im Vakuum vom Lösungsmittel befreit und über eine Kolonne fraktioniert. Man erhält 332g (86 % der Theorie) 2,2-Dimethyl-3-oxo-butyl-methansulfonat vom Siedepunkt 106-120°C/0,12 mm Hg-Säule.

**Beispiel 2**

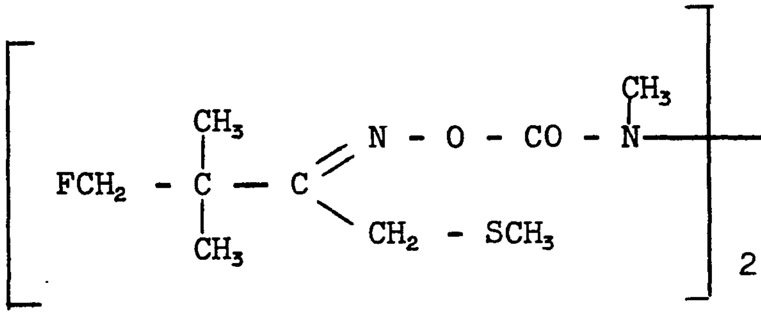

(Verfahren b)

7g (0,039 Mol) 3,3-Dimethyl-4-fluor-1-methylthio-2-hydroximino-butan und 3,68g (0,02 Mol) N,N'-Bis-(fluorcarbonyl)-thio-bis-methylamin werden gemeinsam in 100 ml absolutem Toluol gelöst und bei Zimmertemperatur tropfenweise mit 4g (0,04 Mol) Triethylamin versetzt. Es wird noch 24 Stunden bei dieser Temperatur gerührt, die Toluolphase zweimal mit je 50 ml Wasser gewaschen, über Natriumsulfat getrocknet und eingeengt. Man erhält 8 g (80 % der Theorie) N,N'-Bis-(3,3-dimethyl-4-fluor-1-methylthio-2-oximino-carbonyl-butan)-thio-bis-methylamin als gelbliches, viskoses Oel.

In analoger Weise und entsprechend den Verfahren (a), (b) und (c) können die folgenden Verbindungen der allgemeinen Formel

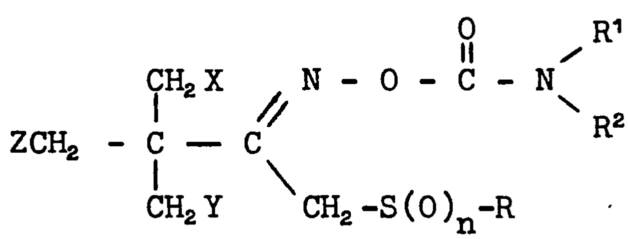 (I)

erhalten werden:

| Bsp. Nr. | X | Y | Z | R | $R^1$ | $R^2$ | n | Schmelzpunkt(°C) |
|---|---|---|---|---|---|---|---|---|
| 3 | H | F | F | $CH_3$ | $CH_3$ | H | 0 | viskoses Oel |
| 4 | F | F | F | $CH_3$ | $CH_3$ | H | 0 | viskoses Oel |
| 5 | H | H | Cl | $CH_3$ | $CH_3$ | H | 0 | viskoses Oel |
| 6 | H | Cl | Cl | $CH_3$ | $CH_3$ | H | 0 | viskoses Oel |
| 7 | H | H | Br | $CH_3$ | $CH_3$ | H | 0 | viskoses Oel |
| 8 | H | Br | Br | $CH_3$ | $CH_3$ | H | 0 | viskoses Oel |
| 9 | H | F | F | $CH_3$ | $CH_3$ | dimer | 0 | viskoses Oel |
| 10 | H | H | Cl | $CH_3$ | $CH_3$ | dimer | 0 | viskoses Oel |

Patentansprüche

1. Substituierte 2-Carbamoyloximino-butane der allgemeinen Formel (I)

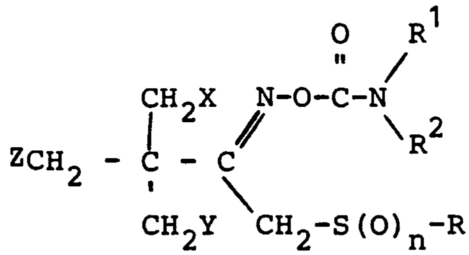

in welcher

R für Alkyl oder Alkylthioalkyl steht,

$R^1$ für Alkyl, Alkoxyalkyl, Alkenyl oder Alkinyl steht,

$R^2$ für Wasserstoff, Alkyl, oder die Gruppe $-SR^3$ steht, wobei

$R^3$ für Alkyl, Halogenalkyl, gegebenenfalls substituiertes Phenyl, Alkoxycarbonyl, die Gruppierung $-N(CH_3)R^4$, sowie für den gleichen Rest steht, an den die Gruppe $-SR^3$ gebunden ist,

$R^4$ für Alkyl, Alkoxycarbonyl oder für gegebenenfalls substituiertes Phenylsulfonyl steht,

X für Wasserstoff oder Halogen steht,

Y für Wasserstoff oder Halogen steht,

Z für Halogen steht und

n für die Zahlen 0, 1 oder 2 steht.

2. Verfahren zur Herstellung der substituierten 2-Carbamoyloximino-butane der Formel (I), dadurch gekennzeichnet, daß man Oxime der Formel (II)

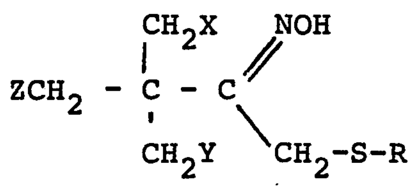

(II)

in welcher

R,X,Y und Z die oben angebene Bedeutung haben,

a) mit Isocyanaten der Formel (III)

$$R^1\text{-N=C=O} \qquad \text{(III)}$$

in welcher

$R^1$ die oben angegebene Bedeutung hat,

in Gegenwart eines Verdünnungsmittels und gegebenenfalls in Gegenwart eines Katalysators umsetzt, oder

b) mit Carbamoylhalogeniden der Formel (IV)

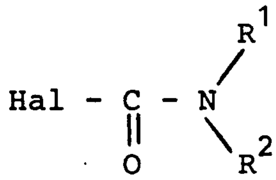

(IV)

in welcher
$R^1$ und $R^2$ die oben angegebene Bedeutung haben und

Hal für Fluor, Chlor oder Brom steht,

in Gegenwart eines Verdünnungsmittels und eines Säurebindemittels umsetzt, oder

c) gegebenenfalls die nach dem Verfahren (a) und (b) erhältlichen Carbamoyloximino-butane der Formel (V)

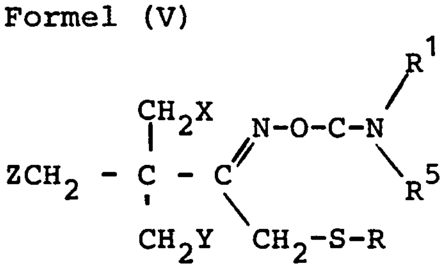

(V)

in welcher
R,$R^1$,X,Y und Z die oben angegebene Bedeutung haben und
$R^5$ für Wasserstoff oder Alkyl mit 1 bis 4 Kohlenstoffatomen steht,

in üblicher Weise oxidiert, wobei je nach verwendetem Oxidationsmittel Verbindungen der Formel (I) mit n= 1 oder n= 2 erhalten werden, oder

d) gegebenenfalls die nach Verfahren (a) und (c) wehältlichen Carbamoyloximino-butane der Formel (Ia)

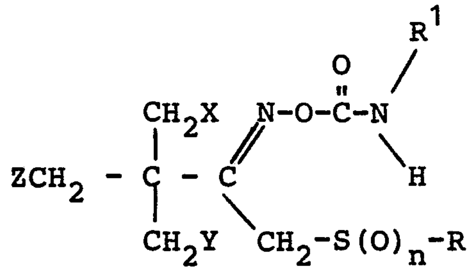

(Ia)

in welcher

$R, R^1, X, Y, Z$ und n die oben angegebene Bedeutung haben,

mit Sulfenchöoriden bzw. -bromiden der Formel (VI)

$$Hal' - S - R^3 \qquad (VI)$$

in welcher

$R^3$ die oben angegebene Bedeutung hat und

Hal' für Chlor oder Brom steht,

in Gegenwart eines Verdünnungsmittels und eines Säurebindemittels umsetzt.

3. Schädlingsbekämpfungsmittel gekennzeichnet durch einen Gehalt an mindestens einem substituierten 2-Carbamoyl-oximino-butanen der Formel (I).

4. Verwendung von substituierten 2-Carbamoyl-oximino-butanen der Formel (I) zur Bekämpfung von Schädlingen.

5. Verfahren zur Bekämpfung von Schädlingen, dadurch gekennzeichnet, daß man substituierten 2-Carbamoyl-oximino-butanen der Formel (I) auf Schädlinge und/oder ihren Lebensraum einwirken läßt.

6. Verfahren zur Herstellung von Schädlingsbekämpfungsmitteln, dadurch gekennzeichnet, daß man substituierte 2-Carbamoyl-oximino-butanen der Formel (I) mit Streckmitteln und/oder oberflächenaktiven Mitteln vermischt.